# EUROPÄISCHE PATENTSCHRIFT

(11) **EP 2 596 812 B1**
(45) Veröffentlichungstag und Bekanntmachung des Hinweises auf die Patenterteilung: **10.06.2015**
(21) Anmeldenummer: 11009254.1
(22) Anmeldetag: 22.11.2011
(51) Int. Cl.: A61L 24/00, A61L 27/16

(54) **Sterilisation von polymerisierbaren Monomeren**
Sterilisation of polymerisable monomers
Stérilisation de monomères polymérisables

(43) Veröffentlichungstag der Anmeldung: 29.05.2013
(73) Patentinhaber: Heraeus Medical GmbH, 61273 Wehrheim (DE)
(72) Erfinder: Vogt, Sebastian, 99092 Erfurt (DE)
(74) Vertreter: Brand, Normen

(56) Entgegenhaltungen:
- EP-A1- 0 364 842
- EP-A2- 2 052 748
- EP-A2- 2 198 893
- WO-A2-2005/021470
- DE-A1- 3 900 862
- DE-A1-102007 019 044
- DE-A1-102008 030 312
- DATABASE EMBASE [Online] ELSEVIER SCIENCE PUBLISHERS, AMSTERDAM, NL; 1969, KUZMENKO L M: "Use of beta propiolactone for sterilization of bone tissue. experimental study (Russian)", XP002673841, Database accession no. EMB-0008186120 & KUZMENKO L M: "Use of beta propiolactone for sterilization of bone tissue. experimental study (Russian)", ORTORTRAVM-PRCJTEZ. 1969, Bd. 30, Nr. 2, 1969, Seiten 59-63,
- DATABASE EMBASE [Online] ELSEVIER SCIENCE PUBLISHERS, AMSTERDAM, NL; 1958, SUTHERLAND T W ET AL: "Graft sterilization. A bacteriological and histological study of the relative merits of ethylene oxide and [beta]-propiolactone as tissue sterilizing agents, with special reference to arterial grafts", XP002673842, Database accession no. EMB-0007659302 & SUTHERLAND T W ET AL: "Graft sterilization. A bacteriological and histological study of the relative merits of ethylene oxide and [beta]-propiolactone as tissue sterilizing agents, with special reference to arterial grafts", BRITISH MEDICAL JOURNAL (1857) 1958, Bd. 5073, 1958, Seiten 734-736, ISSN: 0007-1447
- DATABASE WPI Week 198216 Thomson Scientific, London, GB; AN 1982-31684E XP002673843, & JP 57 042650 A (MITSUBISHI PETROCHEMICAL CO LTD) 10. März 1982 (1982-03-10)
- DATABASE CA [Online] CHEMICAL ABSTRACTS SERVICE, COLUMBUS, OHIO, US; 12. Mai 1984 (1984-05-12), XP002673844, gefunden im STN-International Database accession no. 97:128273 & JP 57 062229 A (MITSUBISHI PETROCHEMICAL CO) 15. April 1982 (1982-04-15)
- DATABASE WPI Week 200915 Thomson Scientific, London, GB; AN 2009-B38425 XP002673845, & KR 2008 0052865 A (LG CHEM LTD) 12. Juni 2008 (2008-06-12)
- DATABASE WPI Week 200454 Thomson Scientific, London, GB; AN 2004-555685 XP002673846, & JP 2004 188679 A (FUJI PHOTO FILM CO LTD) 8. Juli 2004 (2004-07-08)
- DATABASE EPODOC EUROPEAN PATENT OFFICE, THE HAGUE, NL & JP S57 42650 A (MITSUBISHI PETROCHEMICAL CO) 10 March 1982 (1982-03-10)

## Beschreibung

Die Erfindung betrifft ein Verfahren zur Sterilisation eines polymerisierbaren Monomers und Mischungen enthaltend ein polymerisierbares Monomer. Beschrieben werden ein Kit zur Herstellung von Knochenzement enthaltend eine solche Mischung und eine Knochenzementpaste enthaltend eine solche Mischung.

Konventionelle Poly(methylmethacrylat)-Knochenzemente (PMMA-Knochenzemente) sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30).

Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise Suspensionspolymerisation, hergestellt sind, (ii) einen Röntgenopaker und (iii) einen Initiator (zum Beispiel Dibenzoylperoxid). Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Als Alternative zu den konventionellen Pulver-Flüssigkeits-Polymethylmethacrylatknochenzementen wird im Patent DE 102007050762 B3 ein Kit zur Herstellung von Knochenzement vorgeschlagen, der zwei Pasten umfasst. Diese Pasten enthalten jeweils ein polymerisierbares Monomer, wie zum Beispiel ein radikalisch polymerisierbares Methacrylatmonomer, ein in diesem Methacrylatmonomer lösliches Polymer und ein in diesem Methacrylatmonomer unlösliches partikuläres Polymer. In einer dieser Pasten ist zudem ein radikalischer Polymerisationsinitiator enthalten, während die andere Paste einen Polymerisationsaktivator aufweist. Der aus diesen Pasten hergestellte Knochenzement weist als Folge der gewählten Zusammensetzung eine ausreichend hohe Viskosität und Kohäsion auf, um dem Blutungsdruck bis zur Aushärtung standzuhalten. Beim Vermischen der beiden Pasten reagiert der Polymerisationsinitiator mit dem Akzelerator unter Bildung von Radikalen, die die radikalische Polymerisation der Methacrylatmonomere initiieren. Durch die fortschreitende Polymerisation härtet die Paste unter Verbrauch der Methacrylatmonomere aus. Bei den in dem Kit zur Herstellung von Knochenzement enthaltenen Pasten handelt es sich um nichtwässrige Systeme. Daher sind in den Pasten, wen überhaupt, nur Spuren von Wasser enthalten.

PMMA-Knochenzemente stellen Medizinprodukte der Klasse IIb, bei Zusatz von Antibiotika Medizinprodukte der Klasse III dar. Um die Sicherheit der Patienten zu gewährleisten, dürfen die PMMA-Knochenzemente nur im sterilen Zustand in doppeltsteriler Verpackung in den Verkehr gebracht werden dürfen. Bei den herkömmlichen PMMA-Knochenzementen aus einer flüssigen Monomerkomponente und einer Pulverkomponente wird die Pulverkomponente durch Einwirkung von Ethylenoxid sterilisiert. Daneben ist auch die Sterilisation der Pulverkomponente durch Gammabestrahlung üblich.

Das für die Herstellung der Monomerkomponente oft eingesetzte polymerisierbare Monomer Methylmethacrylat ist aufgrund seiner lipophilen und damit denaturierenden Eigenschaften für die meisten vegetativen mikrobiellen Lebensformen biozid. In wasserfreiem Methylmethacrylat können diese Mikroorganismen daher nicht existieren. Neben den vegetativen Formen der Mikroorganismen gibt es jedoch auch generative Formen, wie zum Beispiel Endosporen. Sie stellen generative Überdauerungsformen von Mikroorganismen dar und werden von Gram-positiven Bakterien, besonders der Gattungen *Bacillus* und *Clostridium,* gebildet, um ungünstige Lebensbedingungen überdauern zu können. Endosporen haben im Ruhezustand keinen aktiven Stoffwechsel und besitzen eine mehrschichtige Sporenhülle, die den Sporen-Kern vor Einwirkung von Chemikalien und anderen Umwelteinflüssen weitgehend schützt. Dadurch sind Endosporen extrem widerstandsfähig gegenüber der Einwirkung von Hitze und von Chemikalien (Borick, P. M.: Chemical sterilizers. Adv. Appl. Microbiol. 10 (1968) 291-312; Gould, G. W.: Recent advances in the understanding of resistance and dormacy in bacterial spores. J. Appl. Bacteriol. 42 (1977) 297-309; Gould, G. W.: Mechanisms of resistance and dormancy. p. 173-209. In Hurst, A. and Gould, G. W. (ed.), The bacterial spore. vol. 2 Academic Press, Inc. New York, 1983). Endosporen werden aufgrund ihrer hohen Widerstandsfähigkeit als Bioindikatoren für die Validierung und die Wirksamkeitskontrolle von Sterilisationsprozessen eingesetzt. Es wird dabei davon ausgegangen, dass eine Inaktivierung der Endosporen eine Abtötung aller vegetativen mikrobiellen Lebensformen abbildet. Endosporen von Gram-positiven Bakterien gehören der internationalen Resistenzstufe III an. Zu den Resistenzstufen I gehören nichtsporenbildende Bakterien und vegetative Formen von Sporenbildnern und zur Resistenzstufe II zählen Sporen, die in strömenden Wasserdampf bei 105 °C innerhalb weniger Minuten abgetötet werden. Bei einer Sterilisation müssen nach DAB (Deutsches Arzneimittelbuch) 2008 alle Mikroorganismen der Resistenzstufen I-III abgetötet beziehungsweise irreversibel inaktiviert werden.

Es besteht daher grundsätzlich Bedarf an Verfahren zur effizienten Sterilisation von polymerisierbaren Monomeren.

Verfahren zur Sterilisierung von polymerisierbaren Monomeren sind auf dem Gebiet der Medizinprodukte bekannt.

Zur Sterilisation von Medizinprodukten werden häufig physikalische Sterilisationsverfahren durchgeführt. Dabei sind insbesondere Gammabestrahlung, Elektronenbestrahlung, UV-Bestrahlung, Hitzesterilisation und Autoklavieren mit gespanntem Dampf zu nennen. Nachteilig an diesen Sterilisationsverfahren ist grundsätzlich der zwangsläufig zu betreibende hohe apparative und verfahrenstechnische Aufwand. Eine Sterilisation von polymerisierbaren Monomeren durch diese physikalischen Sterilisationsverfahren scheidet jedoch auch aus anderen Gründen aus: So würde bei Hitzeeinwirkung, bei Gammabestrahlung oder bei Röntgenbestrahlung eine radikalische Polymerisation der polymerisierbaren Monomere ausgelöst werden, was eine unbeabsichtigte, vorzeitige Aushärtung des Knochenzements bewirken würde. Bei einer Dampfsterilisation würde es dagegen zu einer Hydrolyse der polymerisierbaren Monomere kommen, wodurch eine Polymerisation der polymerisierbaren Monomere verhindert würde.

Eine Sterilisierung von polymerisierbaren Monomeren wird oft durch Sterilfiltration und nachfolgende aseptische Abfüllung erreicht. Die aseptische Herstellung von polymerisierbaren Monomeren ist jedoch überaus kostenintensiv. Problematisch ist dabei ferner, dass Viren mittels Sterilfiltration nicht entfernt werden können. Eine Sterilisation von Pasten zur Herstellung von Knochenzement durch Sterilfiltration ist darüber hinaus aufgrund der hohen Viskosität der Pasten und den in den Pasten enthaltenen Röntgenopakern und Füllstoffen nicht möglich.

Neben diesen physikalischen Sterilisationsverfahren werden oft auch chemische Verbindungen zur Sterilisation von Medizinprodukten eingesetzt. Dazu gehören zum Beispiel Ethylenoxid, Formaldehyd, Glutardialdehyd, o-Phthaldialdehyd, Hypochlorid, Chlordioxid, Peressigsäure und Wasserstoffperoxid. Die Verwendung dieser Verbindungen geht jedoch mit signifikanten Nachteilen einher. So wirkt beispielsweise Ethylendioxid nur in Gegenwart von Feuchtigkeit sporozid, so dass dessen Einsatz in Abwesenheit von Wasser nicht zur gewünschten Sterilisationswirkung führt. Ferner liegen Pasten zur Herstellung von Knochenzement üblicherweise in geschlossenen diffusionsdichten Folienbeuteln oder geschlossenen Kunststoffkartuschen vor. In diese Behälter kann jedoch Ethylenoxid nicht eindringen, so dass eine Sterilisation der verpackten Pasten nicht möglich ist. Aldehyde wiederum werden aufgrund ihres Wirkmechanismus üblicherweise als wässrige Lösungen verwendet oder im Fall von Formaldehyd im gasförmigen Zustand. Peressigsäure und Wasserstoffperoxid werden als stark oxidierende Agenzien ebenfalls in Form von wässrigen Lösungen eingesetzt. Zur Sterilisation von Mischungen, die kein Wasser oder Wasser nur in geringen Mengen enthalten dürfen, sind diese Verbindungen daher nicht geeignet. Auf Chlor basierende Verbindungen sind in der Regel sehr wirksame Sterilisierungsmittel. Nachteilig ist jedoch, dass nach der Sterilisation chlorhaltige Folgeprodukte im Medizinprodukt verbleiben.

Aus der pharmazeutischen Industrie ist bekannt, dass wässrige Proteinlösungen, wie zum Beispiel Impfseren, gegenüber oxidierenden Sterilisationsmitteln und verschiedenen physikalischen Sterilisationsverfahren, zum Beispiel der Sterilisation mit Gammastrahlung, sehr empfindlich sind. Aus diesem Grund werden diese wässrigen Proteinlösungen zur Sterilisation oft mit geringen Mengen an dem Acylierungsmittel β-Propiolacton versetzt. Mit β-Propiolacton kann sowohl eine Inaktivierung von Viren als auch von Sporen, insbesondere auch von Endosporen, erreicht werden. Diese Wirkungen treten wohl aufgrund einer Acylierung der Aminogruppen von DNA/RNA oder Proteinen ein. Das als Lösungsmittel enthaltene Wasser ist in der Lage, das β-Propiolacton langsam zu zersetzen, so dass in den wässrigen Proteinlösungen nach kurzer Zeit kein aktives β-Propiolacton mehr enthalten ist. Das Prinzip der Sterilisation von wässrigen Proteinlösungen mittels Acylierungsmitteln, wie β-Propiolacton, basiert darauf, dass in wässrigen Medien eine Quellung der Sporen erfolgen kann. Aufgrund dieser Quellung werden die Doppelwände der Sporen permeabel für diese Acylierungsmittel, so dass diese Acylierungsmittel in die Sporen eindringen und ihre Wirkung entfalten können.

In Mischungen, die polymerisierbares Monomer und, wenn überhaupt, nur geringe Mengen an Wasser enthalten, ist eine Quellung der Sporen jedoch nicht möglich. Daher kann durch Quellung auch keine Vorbereitung der Sporen für ein Eindringen des Acylierungsmittels erfolgen. Folglich scheint eine Sterilisation derartiger Mischungen mittels Acylierungsmitteln, wie β-Propiolacton, nicht möglich zu sein.

Die Patentanmeldung EP-A-2198893 beschreibt die Sterilisation von polymerisierbaren Monomeren für die Herstellung von Knochenzementpasten mit Wasserstoffperoxid.

Der Erfindung liegt daher die Aufgabe zugrunde, ein wirksames Verfahren zur Sterilisation eines polymerisierbaren Monomers bereitzustellen. Durch dieses Verfahren sollen insbesondere Mischungen erhalten werden können, die frei von Endosporen sind. Vorzugsweise sollte dieses Verfahren zur Sterilisation von polymerisierbaren Monomeren in Mischungen geeignet sein, die kein Wasser oder Wasser nur in geringen Mengen, wie zum Beispiel nicht mehr als 2,0 Gewichtsprozent Wasser, mehr bevorzugt nicht mehr als 1,0 Gewichtsprozent Wasser und noch mehr bevorzugt nicht mehr als 0,5 Gewichtsprozent Wasser, bezogen auf das Gesamtgewicht der Mischung, enthalten. Ferner sollten nach der Durchführung der Sterilisation in der Mischung vorzugsweise keine toxischen oder gesundheitsschädlichen Rückstände, wie zum Beispiel chlorhaltige Rückstände, enthalten sein. Darüber hinaus sollten vorzugsweise andere aus dem Stand der Technik bekannten Nachteile vermieden werden.

Weitere Aufgaben, die der Erfindung zugrunde liegen, bestehen in der Bereitstellung einer Mischung (I), die für die Sterilisation gemäß dem erfindungsgemäßen Verfahren verwendbar ist, einer Mischung (II), die bei der Durchführung des erfindungsgemäßen Verfahrens erhalten werden kann, einem Kit zur Herstellung von Knochenzement, der nach der Durchführung des erfindungsgemäßen Verfahrens erhalten werden kann und einer Knochenzementpaste, die nach der Durchführung des erfindungsgemäßen Verfahrens erhalten werden kann.

Diese Aufgaben werden gelöst durch die Gegenstände der unabhängigen Ansprüche. Dementsprechend wird ein Verfahren zur Sterilisation eines polymerisierbaren Monomers bereitgestellt, bei dem eine Mischung (I) hergestellt wird, die wenigstens das polymerisierbare Monomer, eine Verbindung (a) und eine Verbindung (b) enthält, wobei Verbindung (a) ausgewählt ist aus Verbindungen (a1), die durch die allgemeine Formel (I) 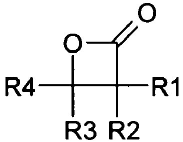 dargestellt sind, wobei R1, R2, R3 und R4 unabhängig voneinander für H, einen substituierten oder unsubstituierten Alkylrest, einen Halogenrest, einen Nitrorest oder einen Cyanorest stehen, Verbindungen (a2), die aus der Gruppe ausgewählt sind, die aus Dimeren der Verbindungen (a1) besteht, und Verbindungen (a3), die aus der Gruppe ausgewählt sind, die aus Dialkyldicarbonaten besteht, und Verbindung (b) aus der Gruppe ausgewählt ist, die aus Wasser und Alkoholen besteht, wobei der Anteil an Verbindung (b) bei nicht mehr als 2 Gewichtsprozent, bezogen auf das Gesamtgewicht der Mischung, liegt.

Die Erfindung stellt außerdem eine Mischung (I) zur Verfügung, enthaltend wenigstens ein polymerisierbares Monomer, eine Verbindung (a) und eine Verbindung (b), wobei Verbindung (a) ausgewählt ist aus Verbindungen (a1), die durch die allgemeine Formel (I) 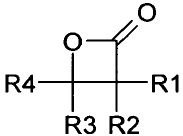 dargestellt sind, wobei R1, R2, R3 und R4 unabhängig voneinander für H, einen substituierten oder unsubstituierten Alkylrest, einen Halogenrest, einen Nitrorest oder einen Cyanorest stehen, Verbindungen (a2), die aus der Gruppe ausgewählt sind, die aus Dimeren der Verbindungen (a1) besteht, und Verbindungen (a3), die aus der Gruppe ausgewählt sind, die aus Dialkyldicarbonaten besteht, und Verbindung (b) aus der Gruppe ausgewählt ist, die aus Wasser und Alkoholen besteht, wobei der Anteil an Verbindung (b) bei nicht mehr als 2 Gewichtsprozent, bezogen auf das Gesamtgewicht von Mischung (I), liegt.

Ferner wird eine Mischung (II) bereitgestellt, enthaltend wenigstens ein polymerisierbares Monomer und eine Verbindung (c), wobei Verbindung (c) aus der Gruppe ausgewählt ist, die aus Alkoholen, Carbonsäuren mit wenigstens drei Kohlenstoffatomen und Estern besteht, und durch Umsetzung einer Verbindung (a) mit einer Verbindung (b) erhältlich ist, wobei Verbindung (a) ausgewählt ist aus Verbindungen (a1), die durch die allgemeine Formel (I) 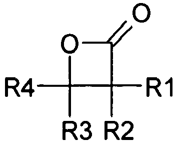 dargestellt sind, wobei R1, R2, R3 und R4 unabhängig voneinander für H, einen substituierten oder unsubstituierten Alkylrest, einen Halogenrest, einen Nitrorest oder einen Cyanorest stehen, Verbindungen (a2), die aus der Gruppe ausgewählt sind, die aus Dimeren der Verbindungen (a1) besteht, und Verbindungen (a3), die aus der Gruppe ausgewählt sind, die aus Dialkyldicarbonaten besteht, und Verbindung (b) aus der Gruppe ausgewählt ist, die aus Wasser und Alkoholen besteht.

Außerdem wird ein Kit zur Herstellung von Knochenzement bereitgestellt, umfassend wenigstens eine Paste A und eine Paste B, wobei wenigstens eine der Pasten A und B eine Mischung II, wie sie hierin beschrieben ist, enthält.

Zudem wird eine Knochenzementpaste zur Verfügung gestellt, enthaltend eine Mischung II, wie sie hierin beschrieben ist.

Die Erfindung beruht zum Teil auf der völlig überraschenden Erkenntnis, dass eine Sterilisation von polymerisierbarem Monomer mit einem Acylierungsmittel gemäß Verbindung (a) in Mischungen auch dann möglich ist, wenn der Anteil an Wasser in diesen Mischungen bei nicht mehr als 2,0 Gewichtsprozent, mehr bevorzugt bei nicht mehr als 1,0 Gewichtsprozent und noch mehr bevorzugt bei nicht mehr als 0,5 Gewichtsprozent, bezogen auf das Gesamtgewicht der Mischung, liegt. Obwohl bei dieser geringen Menge an Wasser keine Quellung der in der Mischung enthaltenen Sporen erfolgen kann, wird überraschenderweise eine effektive Sterilisation des polymerisierbaren Monomers in der Mischung erreicht.

Völlig überraschend ist zudem die Erkenntnis, dass weder Verbindung (a), noch die durch Umsetzung von Verbindung (a) mit Verbindung (b) erhaltenen Produkte die Polymerisation der polymerisierbaren Monomere inhibieren. Dies gilt auch dann, wenn in der Mischung, die dem Verfahren zur Sterilisation unterzogen wird, weitere Bestandteile enthalten sind, wie zum Beispiel Bestandteile einer Paste zur Herstellung von Knochenzement.

Die Erfindung betrifft ein Verfahren zur Sterilisation eines polymerisierbaren Monomers.

Bei dem erfindungsgemäßen Verfahren wird polymerisierbares Monomer erhalten, das durch Sterilität gekennzeichnet ist. Im Rahmen der Erfindung wird unter Sterilität ein von lebensfähigen Mikroorganismen befreiter Zustand bezeichnet. In diesem Zusammenhang kann auf die entsprechende Definition in der EN 556-1:2001 zurückgegriffen werden.

Dem Verfahren zur Sterilisation wird wenigstens ein polymerisierbares Monomer unterzogen.

Unter polymerisierbarem Monomer werden vorzugsweise Verbindungen verstanden, die wenigstens eine polymerisierbare olefinische Bindung aufweisen.

Im erweiterten Sinne werden unter dem Begriff polymerisierbares Monomer auch Makromere mit terminalen Methacrylatgruppen, Acrylatgruppen, Itaconatgruppen, Maleinatgruppen oder Fumaratgruppen verstanden. Diese Makromere können flüssig oder halbflüssig sein. Ferner kann es sich bei den Makromeren um lineare oder verzweigte Verbindungen handeln.

Das erfindungsgemäß eingesetzte polymerisierbare Monomer weist vorzugsweise eine Molmasse von weniger als 1000 g/mol auf. Davon umfasst sind auch polymerisierbare Monomere, die Bestandteil einer Monomermischung sind, wobei wenigstens eines der polymerisierbaren Monomere der Monomermischung eine definierte Struktur mit einer Molmasse von weniger als 1000 g/mol aufweist.

Gemäß einer bevorzugten Ausführungsform ist das polymerisierbare Monomer aus der Gruppe ausgewählt, die aus Methacrylsäureestern (vorzugsweise monofunktionellen und mehrfachfunktionellen Methacrylsäureestern), Acrylsäureestern (vorzugsweise monofunktionellen und mehrfachfunktionellen Acrylsäureestern), Methacrylamid, Methacrylsäure, Acrylsäure, Itaconsäureestern, Itaconsäure, Maleinsäureestern, Maleinsäure, Fumarsäureestern und Fumarsäure besteht. Bei den Methacrylsäureestern und Acrylsäureestern handelt es sich vorzugsweise um Alkylester der Methacylsäure und Acrylsäure. Die Alkylgruppe der Alkylester weist dabei vorzugsweise eine Kettenlänge von 1 - 10 Kohlenstoffatomen, mehr bevorzugt eine Kettenlänge von 1 - 4 Kohlenstoffatomen, noch mehr bevorzugt eine Kettenlänge von 1 - 2 Kohlenstoffatomen und besonders bevorzugt ein Kohlenstoffatom auf.

Gemäß einer besonders bevorzugten Ausführungsform ist das polymerisierbare Monomer aus der Gruppe ausgewählt, die aus Methacrylsäuremethylester, Methacrylamid und Ethylenglykoldimethacrylat besteht.

Zur Sterilisation des polymerisierbaren Monomers wird eine Mischung (I) hergestellt.

Die Mischung (I) ist vorzugsweise selbststerilisierend. Als selbststerilisierend wird eine Mischung vorzugsweise dann bezeichnet, wenn es zur Sterilisation nicht des Zusatzes weiterer Bestandteile oder des Einflusses externer Faktoren, wie zum Beispiel Bestrahlung, bedarf.

Die Mischung (I) enthält neben dem polymerisierbaren Monomer wenigstens eine Verbindung (a).

Bei der Verbindung (a) handelt es sich vorzugsweise um ein Acylierungsmittel. Dieses Acylierungsmittel ist vorzugsweise in der Lage, Aminogruppen von DNA/RNA oder Proteinen zu acylieren.

Die Verbindung (a) ist daher in der Lage, Mikroorganismen abzutöten und so sterilisierende Wirkung zu entfalten.

Die Verbindung (a) ist aus der Gruppe ausgewählt, die aus den hierin beschriebenen Verbindungen (a1), (a2) und (a3) besteht.

Bei der Verbindung (a) kann es sich um eine Verbindung (a1) handeln, die durch die allgemeine Formel (I) 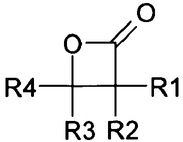 dargestellt ist. In dieser Formel können R1, R2, R3 und R4 unabhängig voneinander für H, einen substituierten oder unsubstituierten Alkylrest, einen Halogenrest, einen Nitrorest oder einen Cyanorest stehen.

Die Stereochemie der Verbindungen (a1) ist nicht weiter eingeschränkt. Vorzugsweise können im Rahmen der Erfindung als Verbindung (I) alle Isomere, die unter die allgemeine Formel (I) fallen, unabhängig von ihrer genauen Konfiguration eingesetzt werden.

Die Alkylreste können unabhängig voneinander substituierte oder unsubstituierte Alkylreste sein. Der wenigstens eine Substituent eines substituierten Alkylrestes ist vorzugsweise aus der Gruppe ausgewählt, die aus Halogenresten, Nitroresten und Cyanoresten besteht.

Die Alkylreste können unabhängig voneinander gesättigte oder ungesättigte Alkylreste sein. Vorzugsweise weist ein ungesättigter Alkylrest wenigstens eine Kohlenstoff-Kohlenstoff-Doppelbindung auf.

Die Alkylreste können unabhängig voneinander verzweigte oder unverzweigte Alkylreste sein. Als Alkylreste R1, R2, R3 und R4 bevorzugt sind unverzweigte Alkylreste.

Die Alkylreste weisen unabhängig voneinander vorzugsweise eine Länge der Hauptkette im Bereich von 1 - 4 Kohlenstoffatomen, mehr bevorzugt eine Länge der Hauptkette im Bereich von 1 - 2 Kohlenstoffatomen und noch mehr bevorzugt ein Kohlenstoffatom auf.

Als Halogenreste kommen in der allgemeinen Formel (I) vorzugsweise Fluorreste, Chlorreste und Bromreste in Betracht. Diese Reste können jeweils unabhängig voneinander für einen oder mehrere der Reste R1, R2 R3 und R4 stehen.

Gemäß einer bevorzugten Ausführungsform stehen die Reste R1, R2, R3 und R4 jeweils für H.

Gemäß einer weiteren bevorzugten Ausführungsform steht der Rest R1 für einen Methylrest und stehen die Reste R2, R3 und R4 für H.

Gemäß einer weiteren bevorzugten Ausführungsform stehen die Reste R1, R2 und R3 für H und steht der Rest R4 für einen Methylrest.

Gemäß noch einer weiteren Ausführungsform stehen die Reste R1 und R3 für H und die Reste R2 und R4 für einen Methylrest.

Gemäß einer besonders bevorzugten Ausführungsform handelt es sich bei der Verbindung (a1) um β-Propiolacton (CAS-Nummer 57-57-8).

Bei der Verbindung (a) kann es sich auch um eine Verbindung (a2) handeln, die ein Dimer aus einer der Verbindungen (a1) ist. Dieses Dimer ist vorzugsweise durch die allgemeine Formel (II) 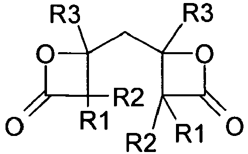 dargestellt, wobei die Reste R1, R2 und R3 unabhängig voneinander die vorstehend definierte Bedeutung aufweisen können.

Die Stereochemie der Verbindungen (a2) ist nicht weiter eingeschränkt. Vorzugsweise können im Rahmen der Erfindung als Verbindung (a2) alle Dimere von Verbindung (a1), insbesondere alle Isomere, die unter die allgemeine Formel (II) fallen, unabhängig von Ihrer genauen Konfiguration eingesetzt werden.

Gemäß einer besonders bevorzugten Ausführungsform stehen die Reste R1, R2 und R3 jeweils für H.

Bei der Verbindung (a) kann es sich auch um eine Verbindung (a3) handeln, die aus der Gruppe ausgewählt ist, die aus Dialkyldicarbonaten besteht.

Die Dialkyldicarbonate können durch die folgende allgemeine Formel (III) dargestellt sein:

R5-O-CO-O-CO-O-R6

Die Reste R5 und R6 können verschieden oder identisch sein. Vorzugsweise sind die Reste R5 und R6 identisch.

Die Reste R5 und R6 können unabhängig voneinander gesättigte Reste oder ungesättigte Reste sein. Ungesättigte Reste weisen vorzugsweise wenigstens eine Kohlenstoff-Kohlenstoff-Doppelbindung auf.

Die Reste R5 und R6 können unabhängig voneinander verzweigte Alkylreste oder unverzweigte Alkylreste sein. Vorzugsweise sind die Reste R5 und R6 unverzweigt.

Die Reste R5 und R6 können unabhängig voneinander substituierte Alkylreste oder unsubstituierte Alkylreste sein. Als Substituenten der Reste R5 und R6 kommen beispielsweise Halogensubstituenten, vorzugsweise Chlorsubstituenten, in Betracht. Vorzugsweise sind die Reste R5 und R6 jedoch unsubstituiert.

Gemäß einer bevorzugten Ausführungsform weisen die Reste R5 und R6 unabhängig voneinander eine Länge der Hauptkette im Bereich von 1 - 8 Kohlenstoffatomen, mehr bevorzugt eine Länge der Hauptkette im Bereich von 1 - 4 Kohlenstoffatomen, noch mehr bevorzugt 1 - 2 Kohlenstoffatome und besonders bevorzugt ein Kohlenstoffatom auf.

Gemäß einer ganz besonders bevorzugten Ausführungsform handelt es sich bei der Verbindung (a) um β-Propiolacton (CAS-Nummer 57-57-8).

Der Anteil von Verbindung (a) liegt vorzugsweise bei wenigstens 0,0001 Gewichtsprozent, mehr bevorzugt bei wenigstens 0,001 Gewichtsprozent, noch mehr bevorzugt bei wenigstens 0,01 Gewichtsprozent und besonders bevorzugt bei wenigstens 0,1 Gewichtsprozent, bezogen auf das Gesamtgewicht von Mischung (I). Vorzugsweise liegt der Anteil von Verbindung (a) bei nicht mehr als 50 Gewichtsprozent, mehr bevorzugt bei nicht mehr als 5 Gewichtsprozent, noch mehr bevorzugt bei nicht mehr als 2 Gewichtsprozent und besonders bevorzugt bei nicht mehr als 0,4 Gewichtsprozent, bezogen auf das Gesamtgewicht von Mischung (I). Der Anteil von Verbindung (a) liegt vorzugsweise im Bereich von 0,0001 - 50 Gewichtsprozent, mehr bevorzugt im Bereich von 0,001 - 5 Gewichtsprozent, noch mehr bevorzugt im Bereich von 0,001 - 2 Gewichtsprozent und besonders bevorzugt im Bereich von 0,1 - 0,4 Gewichtsprozent, bezogen auf das Gesamtgewicht von Mischung (I).

Die Mischung (I), die zur Sterilisation des polymerisierbaren Monomers hergestellt wird, enthält auch eine Verbindung (b).

Die Verbindung (b) ist aus der Gruppe ausgewählt, die aus Wasser und Alkoholen besteht.

Beim Wasser handelt es sich vorzugsweise um doppeltdestilliertes Wasser. Vorzugsweise ist das Wasser pyrogenfrei.

Der Alkohol ist strukturell nicht weiter eingeschränkt.

Vorzugsweise weist der Alkohol eine Länge von 1 - 20 Kohlenstoffatomen in der Hauptkette, mehr bevorzugt eine Länge von 1 - 10 Kohlenstoffatomen in der Hauptkette und noch mehr bevorzugt eine Länge von 1 - 4 Kohlenstoffatomen in der Hauptkette auf.

Der Alkohol kann ein gesättigter oder ein ungesättigter Alkohol sein. Ist der Alkohol ungesättigt, so enthält der Alkohol vorzugsweise wenigstens eine Kohlenstoff-Kohlenstoff-Doppelbindung.

Ferner kann der Alkohol substituiert oder unsubstituiert sein. Der wenigstens eine Substituent des substituierten Alkohols ist vorzugsweise aus der Gruppe ausgewählt, die aus Halogensubstituenten, Cyanosubstituenten und Nitrosubstituenten besteht.

Beim Alkohol kann es sich um einen Monoalkohol oder einen Polyalkohol handeln. Vorzugsweise ist der Alkohol ein Monoalkohol.

Der Alkohol kann ein verzweigter Alkohol oder ein unverzweigter Alkohol sein. Vorzugsweise ist der Alkohol ein unverzweigter Alkohol.

Der Alkohol ist vorzugsweise aus der Gruppe ausgewählt, die aus primären und sekundären Alkoholen besteht.

Nachstehend wird der Alkohol vorzugsweise durch die allgemeine Formel (IV)

R7-OH

beschrieben.

Der Rest R7 ist nicht weiter eingeschränkt. Vorzugsweise handelt es sich bei dem Rest R7 um einen Alkylrest. Dieser Alkylrest weist vorzugsweise 1 - 20 Kohlenstoffatome in der Hauptkette, mehr bevorzugt 1 - 10 Kohlenstoffatome in der Hauptkette, noch mehr bevorzugt 1 - 5 Kohlenstoffatome in der Hauptkette, besonders bevorzugt 1 - 2 Kohlenstoffatome in der Hauptkette und ganz besonders bevorzugt 1 Kohlenstoffatom auf. Der Alkylrest kann gesättigt oder ungesättigt sein. Der Rest R7 kann substituiert oder unsubstituiert sein. Als Substituenten kommen beispielsweise Hydroxylgruppen, Nitrogruppen, Cyanogruppen und Halogene in Betracht. Der Rest R7 kann verzweigt oder unverzweigt sein.

Gemäß einer bevorzugten Ausführungsform ist der primäre Alkohol aus der Gruppe ausgewählt, die aus Methanol, Ethanol, Ethylenglykol, Diethylenglykol, Triethylenglykol, Tetraethylenglykol und Pentaerythrol besteht. Unter den Begriff primärer Alkohol fallen vorzugsweise auch polymerisierbare Monomere mit alkolholischen Hydroxylgruppen, wie zum Beispiel Methacrylsäure-2-hydroxyethylester.

Gemäß einer weiteren bevorzugten Ausführungsform ist der sekundäre Alkohol aus der Gruppe ausgewählt, die aus Isopropanol und Butan-2-ol besteht.

Gemäß einer besonders bevorzugten Ausführungsform ist der Alkohol aus der Gruppe ausgewählt, die aus Methanol, Ethanol, Diethylenglykol, Triethylenglykol und Tetraethylenglykol besteht.

Im Rahmen der Erfindung ist es auch möglich, dass es sich bei dem polymerisierbaren Monomer und Verbindung (b) um dieselbe Verbindung handelt. Beispielsweise kann es sich bei Methacrylsäure-2-hydroxyethylester um das zu sterilisierende polymerisierbare Monomer handeln, das gleichzeitig die Verbindung (b) darstellt.

Der Anteil von Verbindung (b) liegt vorzugsweise bei wenigstens 0,0001 Gewichtsprozent, mehr bevorzugt bei wenigstens 0,001 Gewichtsprozent, noch mehr bevorzugt bei wenigstens 0,01 Gewichtsprozent und besonders bevorzugt bei wenigstens 0,1 Gewichtsprozent, bezogen auf das Gesamtgewicht von Mischung (I). Der Anteil von Verbindung (b) liegt bei nicht mehr als 2,0 Gewichtsprozent, mehr bevorzugt bei nicht mehr als 1,0 Gewichtsprozent, noch mehr bevorzugt bei nicht mehr als 0,5 Gewichtsprozent und besonders bevorzugt bei nicht mehr als 0,4 Gewichtsprozent, bezogen auf das Gesamtgewicht von Mischung (I). Der Anteil von Verbindung (b) liegt vorzugsweise im Bereich von 0,0001 - 2,0 Gewichtsprozent, mehr bevorzugt im Bereich von 0,001 - 1,0 Gewichtsprozent, noch mehr bevorzugt im Bereich von 0,001 - 0,5 Gewichtsprozent und noch mehr bevorzugt im Bereich von 0,1 - 0,4 Gewichtsprozent, bezogen auf das Gesamtgewicht von Mischung (I).

Gemäß einer bevorzugten Ausführungsform erfüllt das Verhältnis der in Mischung (I) enthaltenen Stoffmenge von Verbindung (b), n_{b}, zur in Mischung (I) enthaltenen Stoffmenge von Verbindung (a), nₐ, die Ungleichung n_{b}/nₐ > 0,5, mehr bevorzugt die Ungleichung n_{b}/nₐ > 0,8 und noch mehr bevorzugt die Ungleichung n_{b}/nₐ > 1.

Die Mischung (I) kann zusätzlich zu dem polymerisierbaren Monomer, der Verbindung (a) und der Verbindung (b) gegebenenfalls wenigstens einen weiteren Bestandteil (d) aufweisen.

Beispielsweise ist es erfindungsgemäß, dass es sich bei Mischung (I) um einen Teil eines Medizinprodukts handelt, der neben dem polymerisierbaren Monomer die Verbindungen (a) und (b) enthält. Insbesondere ist es erfindungsgemäß, das Verfahren zur Sterilisation eines polymerisierbaren Monomers an einer Mischung (I) durchzuführen, die neben den Verbindungen (a) und (b) wenigstens einen, vorzugsweise aber alle Inhaltsstoffe enthält, die in einem Bestandteil eines Kits zur Herstellung von Knochenzement enthalten sind, wobei es sich bei dem Bestandteil zum Beispiel um eine Paste handeln kann. Dementsprechend kann es sich bei Mischung (I) auch um eine Mischung handeln, die neben Verbindung (a) und Verbindung (b) die Bestandteile eines in der DE 102007052116, der DE 102007050762 oder der DE 102010005956 beschriebenen Polymethylmethacrylat-Knochenzements enthält. Darüber hinaus kann Mischung (I) auch eine Mischung sein, die neben den Verbindungen (a) und (b) die Bestandteile von anorganischen Knochenzementen oder von polymerisierbaren Dentalwerkstoffen enthält. Bei Mischung (I) kann es sich ferner um eine Mischung handeln, die neben Verbindung (a) und Verbindung (b) die Bestandteile einer Monomerflüssigkeit enthält, die in einem Kit zur Herstellung von Knochenzement (zum Beispiel von Polymethylmethacrylat-Knochenzement) enthalten ist.

Als weitere Bestandteile (d) kommen vorzugsweise Substanzen in Betracht, die aus der Gruppe ausgewählt sind, die aus in dem polymerisierbaren Monomer löslichen Polymeren (d1), in dem polymerisierbaren Monomer unlöslichen Polymeren (d2), radikalischen Polymerisationsinitiatoren (d3), Polymerisationsaktivatoren (d4), Füllstoffen (d5), Farbstoffen (d6), pharmazeutischen Wirkstoffen (d7) und Mischungen davon besteht.

Gemäß einer bevorzugten Ausführungsform ist der weitere Bestandteil (d) ein in dem polymerisierbaren Monomer lösliches Polymer (d1). Bei dem in dem polymerisierbaren Monomer löslichen Polymer (d1) handelt es sich vorzugsweise um ein Polymer mit einer gewichtsmittleren Molmasse von weniger als 500.000 g/mol und mehr bevorzugt um ein Polymer mit einer gewichtsmittleren Molmasse von weniger als 150.000 g/mol. Die Angabe der Molmasse bezieht sich auf die viskosimetrisch bestimmte Molmasse. Das lösliche Polymer (d1) kann vernetzt oder unvernetzt sein und ist vorzugsweise unvernetzt. Das lösliche Polymer (d1) kann ein Homopolymer oder ein Copolymer sein. Vorzugsweise ist das lösliche Polymer (d1) ein Polymer eines Methacrylsäureesters. Gemäß einer besonders bevorzugten Ausführungsform ist das lösliche Polymer (d1) ein Copolymer von Methacrylsäuremethylester. Gemäß einer weiteren besonders bevorzugten Ausführungsform ist das lösliche Polymer (d1) aus der Gruppe ausgewählt, die aus Poly(methacrylsäuremethylester) (PMMA), Poly(methacrylsäureethylester) (PMAE), Poly(methacrylsäurepropylester) (PMAP), Poly(methacrylsäureisopropylester), Poly(methylmethacrylat-co-methylacrylat) und Poly(styrol-co-methylmethacrylat) besteht. Das Polymer (d1) ist in dem polymerisierbaren Monomer löslich. Definitionsgemäß ist das Polymer in dem polymerisierbaren Monomer löslich, wenn das Polymer im polymerisierbaren Monomer bei einer Temperatur von 25°C eine Löslichkeit von wenigstens 25 g/l, mehr bevorzugt eine Löslichkeit von wenigstens 50 g/l und ganz besonders bevorzugt eine Löslichkeit von wenigstens 100 g/l aufweist.

Gemäß einer weiteren bevorzugten Ausführungsform ist der weitere Bestandteil (d) ein in dem polymerisierbaren Monomer unlösliches Polymer (d2). Das unlösliche Polymer (d2) ist vorzugsweise partikulär. Gemäß einer besonders bevorzugten Ausführungsform weist das unlösliche Polymer (d2) eine durchschnittliche Teilchengröße im Bereich von 100 nm - 500 µm auf. Unter durchschnittlicher Teilchengröße wird vorliegend ein Größenbereich verstanden, der von wenigstens 90 Prozent der Teilchen eingenommen wird. Das unlösliche Polymer (d2) weist vorzugsweise eine gewichtsmittlere Molmasse von wenigstens 150.000 g/mol und mehr bevorzugt eine gewichtsmittlere Molmasse von wenigstens 500.000 g/mol auf. Die Angabe der Molmasse bezieht sich auf die viskosimetrisch bestimmte Molmasse. Das unlösliche Polymer (d2) kann vernetzt oder unvernetzt sein und ist vorzugsweise vernetzt. Die Vernetzung erfolgt dabei vorzugsweise mit einer difunktionellen Verbindung. Die difunktionelle Verbindung kann beispielsweise aus der Gruppe ausgewählt sein, die aus Alkylenglykoldimethacrylaten besteht. Als Vernetzter hat sich beispielsweise Ethylenglykoldimethacrylat bewährt. Bei dem unlöslichen Polymer (d2) kann es sich um ein Homopolymer oder um ein Copolymer handeln. Vorzugsweise handelt es sich bei dem unlöslichen Polymer (d2) um ein Polymer eines Methacrylsäureesters. Gemäß einer bevorzugten Ausführungsform ist das unlösliche Polymer (d2) ein Homopolymer oder ein Copolymer eines Methacrylsäurealkylesters. Gemäß einer besonders bevorzugten Ausführungsform ist das unlösliche Polymer (d2) aus der Gruppe ausgewählt, die aus vernetztem Poly(methylmethacrylat-co-methylacrylat) und vernetztem Poly(methylmethacrylat) besteht. Das unlösliche Polymer (d2) ist in dem polymerisierbaren Monomer unlöslich. Definitionsgemäß ist das Polymer in dem polymerisierbaren Monomer unlöslich, wenn das Polymer bei einer Temperatur von 25°C eine Löslichkeit im polymerisierbaren Monomer von weniger als 50 g/l, vorzugsweise von weniger als 25 g/l, mehr bevorzugt von weniger als 10 g/l und noch mehr bevorzugt von weniger als 5 g/l aufweist.

Gemäß noch einer weiteren bevorzugten Ausführungsform ist der weitere Bestandteil (d) ein radikalischer Polymerisationsinitiator (d3).

Gemäß noch einer weiteren bevorzugten Ausführungsform ist der weitere Bestandteil (d) ein Polymerisationsaktivator (d4). Dieser Polymerisationsaktivator kann zusätzlich oder alternativ zu dem radikalischen Polymerisationsinitiator (d3) vorliegen.

Gemäß noch einer bevorzugten Ausführungsform ist der weitere Bestandteil (d) ein Füllstoff (d5). Der Füllstoff (d5) ist vorzugsweise aus der Gruppe ausgewählt, die aus anorganischen Füllstoffen, organischen Füllstoffen, Glas, Metallen und Kohlenstoff besteht. Anorganische Füllstoffe sind vorzugsweise aus der Gruppe ausgewählt, die aus Calciumsulfaten (wie Calciumsulfat, Calciumsulfatdihydrat oder Calciumsulfathemihydrat), Calciumcarbonat, Calciumphosphaten (wie α-Tricalciumphosphat oder β-Tricalciumphosphat), Hydroxylapatit, Bariumsulfat und Zirkoniumdioxid besteht. Organische Füllstoffe sind vorzugsweise aus der Gruppe ausgewählt, die aus unvernetzten Polymerpartikeln, vernetzten Polymerpartikeln und Polymerfasern besteht. Das Glas kann als Füllstoff beispielsweise in Form eines Glaspulvers oder in Form von Glasfasern vorliegen. Das Metall kann vorzugsweise Tantal oder Zirkonium sein.

Gemäß noch einer weiteren bevorzugten Ausführungsform ist der weitere Bestandteil (d) ein Farbstoff (d6).

Gemäß noch einer weiteren bevorzugten Ausführungsform ist der weitere Bestandteil ein pharmazeutischer Wirkstoff (d7). Dieser pharmazeutische Wirkstoff (d7) ist vorzugsweise aus der Gruppe ausgewählt, die aus Antibiotika, Antiinfektiva, Antiseptika, Antiphlogistika und Wachstumsfaktoren besteht.

Die Mischung (I) kann bei Raumtemperatur und einem Druck von 1,013 bar vorzugsweise flüssig oder halbflüssig sein.

Gemäß einer bevorzugten Ausführungsform liegt Mischung (I), die wenigstens das polymerisierbare Monomer, die Verbindung (a) und die Verbindung (b) enthält, in einem Packmittel vor. Dieses Packmittel ist vorzugsweise verschlossen. Vorzugsweise ist das Packmittel diffusionsdicht. Bei dem Packmittel kann es sich beispielsweise um eine Kartusche handeln. Möglich ist auch, dass das Packmittel in einer Kartusche enthalten ist. Die Kartusche kann Teil einer Applikationsvorrichtung für Knochenzementpaste sein. Diese Applikationsvorrichtung kann vorzugsweise eine Mischvorrichtung enthalten. Diese Mischvorrichtung kann zum Vermischen einzelner Komponenten eines Kits zur Herstellung von Knochenzement zu einer Knochenzementpaste geeignet sein.

Als vorteilhaft für das erfindungsgemäße Verfahren zur Sterilisation eines polymerisierbaren Monomers hat sich eine Einwirkzeit von Verbindung (a) auf das polymerisierbare Monomer von wenigstens 20 Minuten und mehr bevorzugt von wenigstens 30 Minuten erwiesen.

Ferner ist vorzugsweise die Temperatur beim erfindungsgemäßen Verfahren zur Sterilisation eines polymerisierbaren Monomers, bei der Verbindung (a) auf das polymerisierbare Monomer einwirkt, vorzugsweise größer oder gleich der Schmelztemperatur des polymerisierbaren Monomers ist.

Die Verbindung (b) weist die Eigenschaft auf, mit Verbindung (a) reagieren zu können. Bei der Umsetzung von Verbindung (a) mit Verbindung (b) werden je nach Struktur der Verbindungen (a) und (b) unterschiedliche Produkte erhalten. Die Umsetzung von Verbindung (a) mit Verbindung (b) erfolgt überraschenderweise in Mischungen, die ein polymerisierbares Monomer enthalten, mit einer vergleichsweise langsamen Reaktionsgeschwindigkeit. Daher entfaltet Verbindung (a) in Mischungen mit Verbindungen (b) zunächst ihre sterilisierende Wirkung, bevor eine Umsetzung mit Verbindung (b) erfolgt. Durch die Umsetzung von Verbindung (a) mit Verbindung (b) kann ferner die sterilisierende Wirkung von Verbindung (a) zeitlich begrenzt werden.

Ein wesentlicher Vorteil des erfindungsgemäßen Verfahrens zur Sterilisation eines polymerisierbaren Monomers besteht darin, dass nach der Herstellung von Mischung (I) die sterilisierende Wirkung ohne weitere externe Einflüsse erfolgt. Ferner ist es zum Beispiel möglich, Mischung (I) in ein geeignetes, vorzugsweise diffusionsdichtes Packmittel einzufüllen. Dabei kommt die Verbindung (a) auch mit der Innenseite des Packmittels in Kontakt, so dass durch die Einwirkung von Verbindung (a) nicht durch das polymerisierbare Monomer und gegebenenfalls weitere in Mischung (I) enthaltene Bestandteile, sondern darüber hinaus auch die Innenseite des Packmittels sterilisiert wird.

Beim erfindungsgemäßen Verfahren zur Sterilisation eines polymerisierbaren Monomers wird eine Mischung (II) erhalten.

Die Mischung (II) weist wenigstens ein polymerisierbares Monomer. Bei diesem polymerisierbaren Monomer handelt es sich vorzugsweise um ein polymerisierbares Monomer gemäß der vorstehenden Beschreibung im Zusammenhang mit Mischung (I).

Die Mischung (II) enthält zudem eine Verbindung (c). Verbindung (c) wird durch Umsetzung der vorstehend beschriebenen Verbindungen (a) und (b) erhalten.

Diese Umsetzung erfolgt vorzugsweise bei Raumtemperatur und ohne dass weitere externe Verfahrensmaßnahmen erforderlich sind.

Bei der Umsetzung der Verbindungen (a) und (b) werden je nach Struktur dieser Verbindungen unterschiedliche Produkte erhalten.

Liegt in Mischung (I) als Verbindung (b) Wasser und als Verbindung (a) eine Verbindung (a1) mit der allgemeinen Formel (I) vor, so kommt es beim erfindungsgemäßen Verfahren unter Lactonring-Öffnung zur Bildung einer Carbonsäure. Diese Carbonsäure kann vorzugsweise durch die allgemeine Formel (V) beschrieben werden

HOOC-CR1 R2-CR3R4-OH,

wobei die Reste R1, R2, R3 und R4 unabhängig voneinander die vorstehend beschriebene Bedeutung haben können.

Liegt in Mischung (I) als Verbindung (b) ein Alkohol mit der allgemeinen Formel (IV) und als Verbindung (a) eine Verbindung (a1) mit der allgemeinen Formel (I) vor, so kommt es beim erfindungsgemäßen Verfahren unter Lactonring-Öffnung zur Bildung eines Esters. Dieser Ester kann vorzugsweise durch die allgemeine Formel (VI) beschrieben werden

R7OOC-CR1 R2-CR3R4-OH,

wobei die Reste R1, R2, R3, R4 und R7 unabhängig voneinander die vorstehend beschriebene Bedeutung haben können.

Liegt in Mischung (I) als Verbindung (b) Wasser und als Verbindung (a) eine Verbindung (a2) mit der allgemeinen Formel (II) vor, so kommt es beim erfindungsgemäßen Verfahren unter Lactonring-Öffnung zur Bildung einer Carbonsäure. Diese Carbonsäure kann vorzugsweise durch die allgemeine Formel (VII) beschrieben werden

HOOC-CR1 R2-CR3(OH)-CH₂-CR₃(OH)-CR1 R2-COOH,

wobei die Reste R1, R2 und R3 unabhängig voneinander die vorstehend beschriebene Bedeutung haben können.

Liegt in Mischung (I) als Verbindung (b) ein Alkohol und als Verbindung (a) eine Verbindung (a2) mit der allgemeinen Formel (II) vor, so kommt es beim erfindungsgemäßen Verfahren unter Lactonring-Öffnung zur Bildung eines Esters. Dieser Ester kann vorzugsweise durch die allgemeine Formel (VIII) beschrieben werden:

R7OOC-CR1R2-CR3(OH)-CH₂-CR₃(OH)-CR1 R2-COOR7,

wobei die Reste R1, R2, R3 und R7 unabhängig voneinander die vorstehend beschriebene Bedeutung haben können.

Liegt in Mischung (I) als Verbindung (b) Wasser und als Verbindung (a) eine Verbindung (a3) mit der allgemeinen Formel (III) vor, so kommt es beim erfindungsgemäßen Verfahren zur Bildung von Kohlendioxid und wenigstens eines Alkohols. Dieser wenigstens eine Alkohol kann vorzugsweise durch die allgemeine Formel (IX)

R5-OH

oder durch die allgemeine Formel (X)

R6-OH

beschrieben werden,
wobei die Reste R5 und R6 unabhängig voneinander die vorstehend beschriebene Bedeutung haben können.

Liegt in Mischung (I) als Verbindung (b) ein Alkohol mit der allgemeinen Formel (IV) und als Verbindung (a) eine Verbindung (a3) mit der allgemeinen Formel (III) vor, so kommt es beim erfindungsgemäßen Verfahren zur Bildung von Kohlendioxid, einem Alkohol und einem Ester. Der Alkohol kann vorzugsweise durch die allgemeine Formel (IX)

R5-OH

oder die allgemeine Formel (X)
R6-OH
beschrieben werden, wobei die Reste R5 und R6 unabhängig voneinander die vorstehend beschriebene Bedeutung haben können.

Der Ester kann vorzugsweise durch die allgemeine Formel (XI)

R7O-CO-OR7

beschrieben werden, wobei der Rest R7 die vorstehend beschriebene Bedeutung haben kann.

Verbindung (c), die aus der Umsetzung von Verbindung (a) mit Verbindung (b) entsteht, ist aus der Gruppe ausgewählt, die aus Alkoholen, Carbonsäuren mit wenigstens drei Kohlenstoffatomen und Estern besteht.

Gemäß einer bevorzugten Ausführungsform ist der Alkohol aus der Gruppe ausgewählt, die aus Verbindungen mit der allgemeinen Formel (V), Verbindungen mit der allgemeinen Formel (VI), Verbindungen mit der allgemeinen Formel (VII), Verbindungen mit der allgemeinen Formel (VIII), Verbindungen mit der allgemeinen Formel (IX) und Verbindungen mit der allgemeinen Formel (X) besteht, wobei die darin enthaltenen Reste R1, R2, R3, R4, R5, R6 und R7 unabhängig voneinander die vorstehend beschriebene Bedeutung haben können.

Die Carbonsäure ist vorzugsweise aus der Gruppe ausgewählt, die aus Hydroxycarbonsäuren besteht. Bei der Hydroxycarbonsäure handelt es sich vorzugsweise um eine β-Hydroxycarbonsäure. Gemäß einer bevorzugten Ausführungsform ist die Carbonsäure aus der Gruppe ausgewählt, die aus Verbindungen mit der allgemeinen Formel (V) und Verbindungen mit der allgemeinen Formel (VII) besteht, wobei die darin enthaltenen Reste R1, R2, R3 und R4 unabhängig voneinander die vorstehend beschriebene Bedeutung haben können.

Der Ester ist vorzugsweise aus der Gruppe ausgewählt, die aus Hydroxyestern und Diestern besteht. Bei dem Hydroxyester handelt es sich vorzugsweise um einen β-Hydroxyester. Der Diester kann vorzugsweise ein Kohlensäurediester sein. Gemäß einer bevorzugten Ausführungsform ist der Ester aus der Gruppe ausgewählt, die aus Verbindungen mit der allgemeinen Formel (VI), Verbindungen mit der allgemeinen Formel (VIII) und Verbindungen mit der allgemeinen Formel (XI) besteht, wobei die darin enthaltenen Reste R1, R2, R3, R4 und R7 unabhängig voneinander die vorstehend beschriebene Bedeutung haben können.

Gemäß einer bevorzugten Ausführungsform ist Verbindung (c) aus der Gruppe ausgewählt, die aus Verbindungen mit der allgemeinen Formel (V), Verbindungen mit der allgemeinen Formel (VI), Verbindungen mit der allgemeinen Formel (VII), Verbindungen mit der allgemeinen Formel (VIII), Verbindungen mit der allgemeinen Formel (IX), Verbindungen mit der allgemeinen Formel (X) und Verbindungen mit der allgemeinen Formel (XI) besteht, wobei die darin enthaltenen Reste R1, R2, R3, R4, R5, R6 und R7 unabhängig voneinander die vorstehend beschriebene Bedeutung haben können.

Gemäß einer besonders bevorzugten Ausführungsform ist Verbindung (c) aus der Gruppe ausgewählt, die aus 3-Hydroxypropionsäure und 3-Hydroxypropionsäureestern besteht. Der Anteil von Verbindung (c) beträgt vorzugsweise wenigstens 0,0001 Gewichtsprozent, mehr bevorzugt wenigstens 0,001 Gewichtsprozent, noch mehr bevorzugt wenigstens 0,01 Gewichtsprozent und besonders bevorzugt wenigstens 0,1 Gewichtsprozent, bezogen auf das Gesamtgewicht von Mischung (II). Vorzugsweise liegt der Anteil von Verbindung (c) vorzugsweise bei nicht mehr als 5,0 Gewichtsprozent, mehr bevorzugt bei nicht mehr als 2,0 Gewichtsprozent, noch mehr bevorzugt bei nicht mehr als 1,0 Gewichtsprozent und besonders bevorzugt bei nicht mehr als 0,5 Gewichtsprozent, bezogen auf das Gesamtgewicht von Mischung (II). Der Anteil von Verbindung (c) liegt vorzugsweise im Bereich von 0,0001 - 5,0 Gewichtsprozent, mehr bevorzugt im Bereich von 0,001 - 2,0 Gewichtsprozent, noch mehr bevorzugt im Bereich von 0,01 - 1,0 Gewichtsprozent und besonders bevorzugt im Bereich von 0,1 - 0,5 Gewichtsprozent, bezogen auf das Gesamtgewicht von Mischung (II). Die Mischung (II) kann zusätzlich zu dem polymerisierbaren Monomer und der Verbindung (c) gegebenenfalls weitere Bestandteile (d) aufweisen. Bei diesen weiteren Bestandteilen (d) handelt es sich vorzugsweise um die weiteren Bestandteile (d) gemäß der vorstehenden Beschreibung im Zusammenhang mit Mischung (I).

Gemäß einer bevorzugten Ausführungsform liegt Mischung (II), die wenigstens das polymerisierbare Monomer und die Verbindung (c) enthält, in einem Packmittel vor. Dieses Packmittel ist vorzugsweise verschlossen. Vorzugsweise ist das Packmittel diffusionsdicht. Bei dem Packmittel kann es sich beispielsweise um eine Kartusche handeln. Möglich ist auch, dass das Packmittel in einer Kartusche enthalten ist. Die Kartusche kann Teil einer Applikationsvorrichtung für Knochenzementpaste sein. Diese Applikationsvorrichtung kann vorzugsweise eine Mischvorrichtung enthalten. Diese Mischvorrichtung kann zum Vermischen einzelner Komponenten eines Kits zur Herstellung von Knochenzement zu einer Knochenzementpaste geeignet sein. Bereitgestellt wird ausserdem ein Kit zur Herstellung von Knochenzement.

Dieser Kit zur Herstellung von Knochenzement umfasst wenigstens eine Paste A und eine Paste B. Wenigstens eine der Pasten A und B enthält eine der vorstehend beschriebenen Mischungen (II). Vorzugsweise enthalten Paste A und Paste B jeweils eine der vorstehend beschriebenen Mischungen (II), wobei sich Paste A in wenigstens einem ihrer Bestandteile von Paste B unterscheidet.

Die Pasten A und B können neben dem polymerisierbaren Monomer und Verbindung (c) wenigstens einen weiteren Bestandteil enthalten. Dieser wenigstens eine weitere Bestandteil kann aus der Gruppe der vorstehend beschriebenen weiteren Bestandteile (d) ausgewählt sein. Gemäß einer bevorzugten Ausführungsform weist Paste A wenigstens ein polymerisierbares Monomer, eine Verbindung (c) und ein in dem polymerisierbaren Monomer lösliches Polymer (d1) und Paste B wenigstens ein polymerisierbares Monomer, eine Verbindung (c) und ein in dem polymerisierbaren Monomer lösliches Polymer (d1) auf. Vorzugsweise ist in wenigstens einer der Pasten A und B zudem ein in dem polymerisierbaren Monomer unlösliches Polymer (d2) enthalten. Es ist auch möglich, dass ein in dem polymerisierbaren Monomer unlösliches Polymer (d2) in Paste A und Paste B enthalten ist. Darüber hinaus ist in wenigstens einer der Pasten A und B vorzugsweise wenigstens ein radikalischer Polymerisationsinitiator (d3) enthalten. Dieser radikalische Polymerisationsinitiator (d3) ist dabei vorzugsweise in der Paste enthalten, in der auch das in dem polymerisierbaren Monomer unlösliche Polymer (d2) enthalten ist. Vorzugsweise weist ferner wenigstens eine der Pasten A und B einen Polymerisationsaktivator (d4) auf. Zudem ist vorzugsweise in wenigstens einer der Pasten A und B ein pharmazeutischer Wirkstoff (d7) enthalten.

Gemäß einer besonders bevorzugten Ausführungsform liegen in dem Kit zur Herstellung von Knochenzement Paste A in einem ersten Packmittel und Paste B in einem zweiten Packmittel vor. Diese Packmittel sind vorzugsweise räumlich voneinander getrennt. Vorzugsweise sind diese Packmittel verschlossen. Ferner sind diese Packmittel vorzugsweise diffusionsdicht. Bei den Packmitteln kann es sich beispielsweise um Kartuschen handeln. Möglich ist auch, dass die Packmittel in Kartuschen enthalten sind. Diese Kartuschen können Teil einer Applikationsvorrichtung für Knochenzementpaste sein. Diese Applikationsvorrichtung kann vorzugsweise eine Mischvorrichtung enthalten. Diese Mischvorrichtung kann zum Vermischen einzelner Komponenten eines Kits zur Herstellung von Knochenzement zu einer Knochenzementpaste geeignet sein.

Beschrieben wird zudem eine Knochenzementpaste. Unter Knochenzementpaste wird hierin eine Paste verstanden, die einem Patienten appliziert werden und selbständig aushärten kann.

Diese Knochenzemenpaste enthält eine Mischung (II), wie sie vorstehend definiert ist.

Gemäß einer bevorzugten Ausführungsform enthält die Knochenzementpaste wenigstens einen weiteren Bestandteil. Dieser wenigstens eine weitere Bestandteil kann aus der Gruppe der vorstehend beschriebenen weiteren Bestandteile (d) ausgewählt sein.

Gemäß einer besonders bevorzugten Ausführungsform weist die Knochenzementpaste erfindungsgemäß neben dem polymerisierbaren Monomer und Verbindung (c) wenigstens ein in dem polymerisierbaren Monomer lösliches Polymer (d1), ein in dem polymerisierbaren Monomer unlösliches Polymer (d2), gegebenenfalls einen radikalischen Polymerisationsinitiator (d3), gegebenenfalls einen Polymerisationsaktivator (d4) und gegebenenfalls einen pharmazeutischer Wirkstoff (d7) auf.

Die Erfindung wird durch nachstehende Beispiele näher erläutert, ohne jedoch die Erfindung zu beschränken.

### BEISPIELE:

### BEISPIEL 1:

Es wurden in fünf 50 ml-Schraubdeckeldosen jeweils 20 g Methylmethacrylat (Sigma-Aldrich, stabilisiert durch Hydrochinon) eingewogen. Nach der Zugabe von 14 µl destilliertem Wasser wurden in jede der Kunststoffflaschen 10 µl einer 40 %igen ethanolischen Sporensuspension von *Bacillus subtilis* ATCC9357 eingebracht. Danach wurden 17 µl (20 mg) ß-Propiolacton zugesetzt. Die Sachraubdeckeldosen wurden verschlossen und nach kurzem intensiven Schütteln sieben Tage bei 23 °C gelagert.

### BEISPIEL 2:

Es wurden in fünf 50 ml-Schraubdeckeldosen jeweils 20 g Methylmethacrylat (Sigma-Aldrich, stabilisiert durch Hydrochinon) eingewogen. Nach der Zugabe von 35 µl destilliertem Wasser wurden in jede der Kunststoffflaschen 10 µl einer 40 %igen ethanolischen Sporensuspension von *Bacillus subtilis* ATCC9357 eingebracht. Danach wurden 34 µl (40 mg) ß-Propiolacton zugesetzt. Die Sachraubdeckeldosen wurden verschlossen und nach kurzem intensiven Schütteln sieben Tage bei 23 °C gelagert.

### BEISPIEL 3:

Es wurden in fünf 50 ml-Schraubdeckeldosen jeweils 20 g Methylmethacrylat (Sigma-Aldrich, stabilisiert durch Hydrochinon) eingewogen. Nach der Zugabe von 20 µl Methanol wurden in jede der Kunststoffflaschen 10 µl einer 40 %igen ethanolischen Sporensuspension von *Bacillus subtilis* ATCC9357 eingebracht. Danach wurden 17 µl (20 mg) β-Propiolacton zugesetzt. Die Sachraubdeckeldosen wurden verschlossen und nach kurzem intensiven Schütteln sieben Tage bei 23 °C gelagert.

### BEISPIEL 4:

Es wurden in fünf 50 ml-Schraubdeckeldosen jeweils 20 g Methylmethacrylat (Sigma-Aldrich, stabilisiert durch Hydrochinon) eingewogen. Nach der Zugabe von 34 µl destilliertem Wasser wurden in jede der Kunststoffflaschen 10 µl einer 40 %igen ethanolischen Sporensuspension von *Bacillus subtilis* ATCC9357 eingebracht. Danach wurden 34 µl (40 mg) Dimethyldicarbonat zugesetzt. Die Sachraubdeckeldosen wurden verschlossen und nach kurzem intensiven Schütteln sieben Tage bei 23 °C gelagert.

### VERGLEICHSBEISPIEL 1:

Es wurden in fünf 50 ml-Schraubdeckeldosen jeweils 20 g Methylmethacrylat (Sigma-Aldrich, stabilisiert durch Hydrochinon) eingewogen. In jede der Kunststoffflaschen wurden dann 10 µl einer 60 %igen ethanolischen Sporensuspension von *Bacillus subtilis* ATCC9357 eingebracht. Die Sachraubdeckeldosen wurden verschlossen und nach kurzem intensiven Schütteln sieben Tage bei 23 °C gelagert.

### BEISPIEL 5:

Es wurden in fünf 50 ml-Schraubdeckeldosen jeweils 8,0 g Methylmethacrylat (Sigma-Aldrich, stabilisiert durch Hydrochinon) eingewogen. Nach der Zugabe von 14 µl destilliertem Wasser wurden in jede der Kunststoffflaschen 10 µl einer 60 %igen ethanolischen Sporensuspension von *Bacillus subtilis* ATCC9357 und 17 µl (20 mg) ß-Propiolacton eingebracht. Anschließend wurde zur Homogenisierung kurz geschüttelt. Danach wurde zu dieser Zubereitung pro Schraubdeckeldose jeweils ein Gemisch aus 1,0 g Zirkoniumdioxid, 5,5 g eines lineraren Polymethylmethacrylat-co-methylacrylats und 5,5 g eines vernetzten Polymethylmethacrylats gegeben. Es bildete sich eine Paste aus. Danach wurde kurz geschüttelt. Die Mischungen wurden dann sieben Tage bei 23 °C gelagert.

### BEISPIEL 6:

Es wurden in fünf 50 ml-Schraubdeckeldosen jeweils 8,0 g Methylmethacrylat (Sigma-Aldrich, stabilisiert durch Hydrochinon) eingewogen. Nach der Zugabe von 35 µl destilliertem Wasser wurden in jede der Kunststoffflaschen 10 µl einer 60 %igen ethanolischen Sporensuspension von *Bacillus subtilis* ATCC9357 und 35 µl (40 mg) ß-Propiolacton eingebracht. Anschließend wurde zur Homogenisierung kurz geschüttelt. Danach wurde zu dieser Zubereitung pro Schraubdeckeldose jeweils ein Gemisch aus 1,0 g Zirkoniumdioxid, 5,5 g eines lineraren Polymethylmethacrylat-co-methylacrylats und 5,5 g eines vernetzten Polymethylmethacrylats gegeben. Es bildete sich eine Paste aus. Danach wurde kurz geschüttelt. Die Mischungen wurden dann sieben Tage bei 23 °C gelagert.

### BEISPIEL 7:

Es wurden in fünf 50 ml-Schraubdeckeldosen jeweils 8,0 g Methylmethacrylat (Sigma-Aldrich, stabilisiert durch Hydrochinon) eingewogen. Nach der Zugabe von 20 µl Methanol wurden in jede der Kunststoffflaschen 10 µl einer 60 %igen ethanolischen Sporensuspension von *Bacillus subtilis* ATCC9357 und 35 µl (40 mg) ß-Propiolacton eingebracht. Anschließend wurde zur Homogenisierung kurz geschüttelt. Danach wurde zu dieser Zubereitung pro Schraubdeckeldose jeweils ein Gemisch aus 1,0 g Zirkoniumdioxid, 5,5 g eines lineraren Polymethylmethacrylat-co-methylacrylats und 5,5 g eines vernetzten Polymethylmethacrylats gegeben. Es bildete sich eine Paste aus. Danach wurde kurz geschüttelt. Die Mischungen wurden dann sieben Tage bei 23 °C gelagert.

### VERGLEICHSBEISPIEL 2:

Es wurden in fünf 50 ml-Schraubdeckeldosen jeweils 8,0 g Methylmethacrylat (Sigma-Aldrich, stabilisiert durch Hydrochinon) eingewogen. In jede der Kunststoffflasche wurden dann 10 µl einer 60 %igen ethanolischen Sporensuspension von *Bacillus subtilis* ATCC9357 eingebracht. Anschließend wurde zur Homogenisierung kurz geschüttelt. Danach wurde zu dieser Zubereitung pro Schraubdeckeldose jeweils ein Gemisch aus 1,0 g Zirkoniumdioxid, 5,5 g eines lineraren Polymethylmethacrylat-co-methylacrylats und 5,5 g eines vernetzten Polymethylmethacrylats gegeben. Danach wurde kurz geschüttelt. Es bildete sich eine Paste aus. Die Mischungen wurden dann sieben Tage bei 23 °C gelagert.

### AUSWERTUNG DER BEISPIELE 1 - 7 UND DER VERGLEICHSBEISPIELE 1 UND 2:

Zur Auswertung wurden die in den Beispielen und Vergleichsbeispielen erhaltenen Mischungen auf Sterilität überprüft.

Hierzu wurden nach sieben Tagen Inkubation jeweils zwei Proben pro Schraubdeckeldose entnommen. Die Proben wurden im Anschluss für 14 Tage bebrütet und danach gemäß ISO11737 Teil 2 auf Sterilität untersucht.

Die Ergebnisse sind in Tabelle 1 dargestellt:

**Tabelle 1: Untersuchung der Mischungen der Beispiele 1 - 7 und der Vergleichsbeispiele 1 und 2 auf Sterilität nach der ISO 11737 Teil 2.**

| **Beispiel** | **Anzahl sterile Proben** | **Anzahl unsterile Proben** |
|---|---|---|
| 1 | 8 | 2 |
| 2 | 10 | 0 |
| 3 | 10 | 0 |
| 4 | 10 | 0 |
| Vergleichsbeispiel 1 | 2 | 8 |
| 5 | 10 | 0 |
| 6 | 10 | 0 |
| 7 | 10 | 0 |
| Vergleichsbeispiel 2 | 2 | 8 |

### BEISPIEL 8:

Es wurden im Folgenden eine Paste A und eine Paste B durch Vermischung der Rohstoffe in separaten Schraubdeckeldosen hergestellt.

| Paste A | |
|---|---|
| Rohstoff | Einwaage |
| 1-Cyclohexyl-5-ethyl-barbitursäure | 2,0 g |
| Methacrylamid | 0,4 g |
| Methylmethacrylat | 18,9 g |
| Lineares, in Methylmethacrylat lösliches Polymethylmethacrylat-co-methylacrylat (Molmasse < 500.000 g/mol) | 6,2 g |
| Vernetztes Polymethylmethacrylat (Siebfraktion < 100 µm) | 15,5 g |
| 2,4-Di-t-butyl-4-methyl-phenol | 20 mg |
| ß-Propiolacton | 86 mg |
| Wasser | 86 mg |
| | |

| Paste B | |
|---|---|
| Rohstoff | Einwaage |
| Lithiumchlorid | 40 mg |
| 2,4-Di-t-butyl-4-methyl-phenol | 35 mg |
| Methylmethacrylat | 18,9 g |
| Lineares, in Methylmethacrylat lösliches Polymethylmethacrylat-co-methylacrylat (Molmasse < 500.000 g/mol) | 16,9 g |
| Grünlack | 50 mg |
| Kupfer(II)-hydroxid | 2 mg |
| ß-Propiolacton | 86 mg |
| Wasser | 86 mg |

Die beiden Pasten wurden über sieben Tage bei Raumtemperatur gelagert. Es wurden dann 3,5 g der Paste A und 3,5 g der Paste B entnommen und intensiv miteinander verknetet. Es entstand ein grüner, klebfreier Zementteig. Der Teig wurde dann weiter mit der Hand geknetet. Nach etwa 2 Minuten und 40 Sekunden war das Einsetzen der Polymerisation durch Wärmefreisetzung spürbar. Nach 4 Minuten und 40 Sekunden war das Ende der Verarbeitbarkeit erreicht. Nach etwa 6 Minuten war der Zementteig ausgehärtet.

### BEISPIEL 9:

Es wurden im Folgenden eine Paste A und eine Paste B hergestellt.

| Paste A | |
|---|---|
| Rohstoff | Einwaage |
| 1-Cyclohexyl-5-ethyl-barbitursäure | 2,0 g |
| Methacrylamid | 0,4 g |
| Methylmethacrylat | 18,9 g |
| Lineares, in Methylmethacrylat lösliches Polymethylmethacrylat-co-methylacrylat (Molmasse < 500.000 g/mol) | 6,2 g |
| Vernetztes Polymethylmethacrylat (Siebfraktion < 100 µm) | 15,5 g |
| 2,4-Di-t-butyl-4-methyl-phenol | 20 mg |
| ß-Propiolacton | 86 mg |
| Wasser | 86 mg |
| | |

| Paste B | |
|---|---|
| Rohstoff | Einwaage |
| Aliquat 336 (Trioctylmethylammoniumchlorid) | 60 mg |
| 2,4-Di-t-butyl-4-methyl-phenol | 35 mg |
| Methylmethacrylat | 18,9 g |
| Lineares, in Methylmethacrylat lösliches Polymethylmethacrylat-co-methylacrylat (Molmasse < 500.000 g/mol) | 16,9 g |
| Grünlack | 50 mg |
| Kupfer(II)hydroxid | 2 mg |
| ß-Propiolacton | 86 mg |
| Wasser | 86 mg |

Die beiden Pasten wurden bei Raumtemperatur über einen Zeitraum von sieben Tagen gelagert. Es wurden dann 3,5 g der Paste A und 3,5 g der Paste B entnommen und intensiv miteinander verknetet. Es entstand ein grüner, klebfreier Zementteig. Der Teig wurde dann weiter mit der Hand geknetet. Nach etwa 3 Minuten war eine Wärmefreisetzung spürbar. Nach 4 Minuten 40 Sekunden war das Ende der Verarbeitbarkeit erreicht. Nach etwa 6 Minuten und 20 Sekunden war der Zementteig ausgehärtet.

### BEISPIEL 10:

Es wurden im Folgenden eine Paste A und eine Paste B hergestellt.

| Paste A | |
|---|---|
| Rohstoff | Einwaage |
| 1-Cyclohexyl-5-ethyl-barbitursäure | 2,0 g |
| Methacrylamid | 0,4 g |
| Methylmethacrylat | 18,9 g |
| Lineares, in Methylmethacrylat lösliches Polymethylmethacrylat-co-methylacrylat (Molmasse < 500.000 g/mol) | 11,5 g |
| Vernetztes Polymethylmethacrylat (Siebfraktion < 100 µm) | 7,7 g |
| 2,4-Di-t-butyl-4-methyl-phenol | 20 mg |
| ß-Propiolacton | 86 mg |
| Wasser | 86 mg |

| Paste B | |
|---|---|
| Rohstoff | Einwaage |
| Aliquat 336 (Trioctylmethylammoniumchlorid) | 60 mg |
| 2,4-Di-t-butyl-4-methyl-phenol | 35 mg |
| Methylmethacrylat | 18,9 g |
| Lineares, in Methylmethacrylat lösliches Polymethylmethacrylat-co-methylacrylat (Molmasse < 500.000 g/mol) | 11,5 g |
| Vernetztes Polymethylmethacrylat (Siebfraktion < 100 µm) | 7,7 g |
| Grünlack | 50 mg |
| Kupfer(II)hydroxid | 2 mg |
| ß-Propiolacton | 86 mg |
| Wasser | 86 mg |

Die beiden Pasten wurden über sieben Tage bei Raumtemperatur gelagert. Es wurden dann 3,5 g der Paste A und 3,5 g der Paste B entnommen und intensiv miteinander verknetet. Es entstand ein grüner, klebfreier Zementteig. Der Teig wurde dann weiter mit der Hand geknetet. Nach 5 Minuten war das Ende der Verarbeitung erreicht. Nach etwa 6 Minuten war der Zementteig ausgehärtet.

## Patentansprüche

1. Verfahren zur Sterilisation eines polymerisierbaren Monomers, bei dem eine Mischung (I) hergestellt wird, die wenigstens das polymerisierbare Monomer, eine Verbindung (a) und eine Verbindung (b) enthält, wobei Verbindung (a) ausgewählt ist aus Verbindungen (a1), die durch die allgemeine Formel (I) 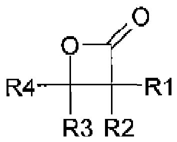 dargestellt sind, wobei R1, R2, R3 und R4 unabhängig voneinander für H, einen substituierten oder unsubstituierten Alkylrest, einen Halogenrest, einen Nitrorest oder einen Cyanorest stehen, Verbindungen (a2), die aus der Gruppe ausgewählt sind, die aus Dimeren der Verbindungen (a1) besteht, und Verbindungen (a3), die aus der Gruppe ausgewählt sind, die aus Dialkyldicarbonaten besteht, und Verbindung (b) aus der Gruppe ausgewählt ist, die aus Wasser und Alkoholen besteht, wobei der Anteil an Verbindung (b) bei nicht mehr als 2 Gewichtsprozent, bezogen auf das Gesamtgewicht von Mischung (I), liegt.

2. Verfahren nach Anspruch 1, **dadurch gekennzeichnet, dass** die Verbindung (a) β-Propiolacton ist.

3. Verfahren nach einem der vorangegangenen Ansprüche, **dadurch gekennzeichnet dass** das Verhältnis der in Mischung (I) enthaltenen Stoffmenge von Verbindung (b), n_{b}, zur in Mischung (I) enthaltenen Stoffmenge von Verbindung (a), nₐ, die Ungleichung n_{b}/nₐ > 0,5, erfüllt.

4. Mischung (I) enthaltend wenigstens ein polymerisierbares Monomer, eine Verbindung
(a) und eine Verbindung (b), wobei Verbindung (a) ausgewählt ist aus Verbindungen (a1), die durch die allgemeine Formel (I) 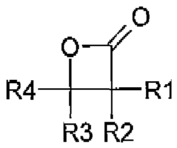 dargestellt sind, wobei R1, R2, R3 und R4 unabhängig voneinander für H, einen substituierten oder unsubstituierten Alkylrest, einen Halogenrest, einen Nitrorest oder einen Cyanorest stehen, Verbindungen (a2), die aus der Gruppe ausgewählt sind, die aus Dimeren der Verbindungen (a1) besteht, und Verbindungen (a3), die aus der Gruppe ausgewählt sind, die aus Dialkyldicarbonaten besteht, und Verbindung (b) aus der Gruppe ausgewählt ist, die aus Wasser und Alkoholen besteht, wobei der Anteil an Verbindung (b) bei nicht mehr als 2 Gewichtsprozent, bezogen auf das Gesamtgewicht von Mischung (I), liegt.

5. Mischung nach Anspruch 4, **dadurch gekennzeichnet, dass** die Verbindung (a) β-Propiolacton ist.

6. Mischung nach einem der Ansprüche 4, **dadurch gekennzeichnet, dass** die Mischung ein in dem polymerisierbaren Monomer lösliches Polymer enthält.

7. Mischung nach einem der Ansprüche 4 - 6, **dadurch gekennzeichnet, dass** die Mischung ein in dem polymerisierbaren Monomer unlösliches Polymer enthält.

## Claims

1. Method for sterilisation of a polymerisable monomer, in which a mixture (I) containing at least the polymerisable monomer, a compound (a) and a compound (b) is produced, whereby
compound (a) is selected from compounds (a1) represented by general formula (I) 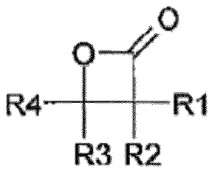 whereby R1, R2, R3, and R4, independent of each other, are H, a substituted or non-substituted alkyl residue, a halogen residue, a nitro residue or a cyano residue, compounds (a2), which are selected from the group consisting of dimers of compounds (a1), and compounds (a3), which are selected from the group consisting of dialkyldicarbonates, and compound (b) is selected from the group consisting of water and alcohols, whereby the fraction of compound (b) is no more than 2 % by weight, based on the total weight of mixture (I).

2. Method according to claim 1, **characterised in that** compound (a) is ß-propiolactone.

3. Method according to any one of the preceding claims, **characterised in that** the ratio of the amount of compound (b), n_{b}, present in mixture (I) and the amount of substance (a), nₐ, present in mixture (I) is described by the inequation n_{b}/nₐ > 0.5.

4. Mixture (I) containing at least a polymerisable monomer, a compound (a) and a compound (b), whereby
compound (a) is selected from compounds (a1) represented by general formula (I) 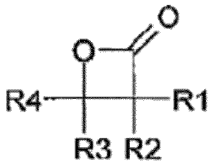 whereby R1, R2, R3, and R4, independent of each other, are H, a substituted or non-substituted alkyl residue, a halogen residue, a nitro residue or a cyano residue, compounds (a2), which are selected from the group consisting of dimers of compounds (a1), and compounds (a3), which are selected from the group consisting of dialkyldicarbonates, and compound (b) is selected from the group consisting of water and alcohols, whereby the fraction of compound (b) is no more than 2 % by weight, based on the total weight of mixture (I).

5. Method according to claim 4, **characterised in that** compound (a) is ß-propiolactone.

6. Mixture according to any one of the claims 4, **characterised in that** the mixture contains a polymer that is soluble in said polymerisable monomer.

7. Mixture according to any one of the claims 4-6, **characterised in that** the mixture contains a polymer that is insoluble in said polymerisable monomer.

## Revendications

1. Procédé de stérilisation d'un monomère polymérisable, lors duquel un mélange (I) est fabriqué, lequel contient au moins le monomère polymérisable, un composé (a) et un composé (b), dans lequel
le composé (a) est sélectionné parmi des composés (a1) qui sont représentés par la formule générale (I) 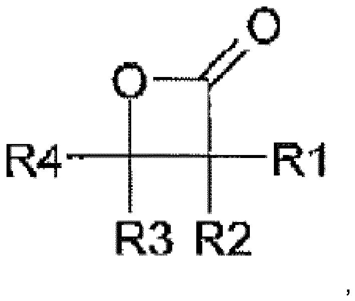 dans laquelle R1, R2, R3 et R4 représentent indépendamment les uns des autres H, un radical alkyle substitué ou non substitué, un radical halogène, un radical nitré ou un radical cyano, des composés (a2) qui sont sélectionnés parmi le groupe qui se compose de dimères des composés (a1) et des composés (a3) qui sont sélectionnés parmi le groupe qui se compose de dicarbonates dialkyliques, et le composé (b) est sélectionné parmi le groupe qui se compose d'eau et d'alcools, dans lequel la proportion en composé (b) se situe à pas plus de 2 pour cent en poids, par rapport au poids total du mélange (I).

2. Procédé selon la revendication 1, **caractérisé en ce que** le composé (a) est une β-propiolactone.

3. Procédé selon une des revendications précédentes, **caractérisé en ce que** le rapport de la quantité de substance contenue dans le mélange (I) de composé (b), n_{b}, sur la quantité de substance contenue dans le mélange (I) de composé (a), nₐ, satisfait l'inéquation n_{b}/nₐ > 0,5.

4. Mélange (I) contenant au moins un monomère polymérisable, un composé (a) et un composé (b),
dans lequel le composé (a) est sélectionné parmi des composés (a1) qui sont représentés par la formule générale (I) 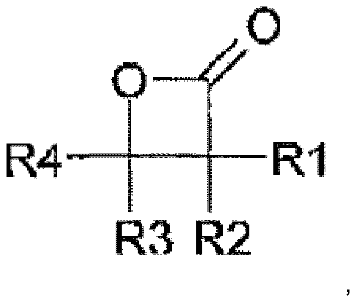 dans laquelle R1, R2, R3 et R4 représentent indépendamment les uns des autres H, un radical alkyle substitué ou non substitué, un radical halogène, un radical nitré ou un radical cyano, des composés (a2) qui sont sélectionnés parmi le groupe qui se compose de dimères des composés (a1) et des composés (a3) qui sont sélectionnés parmi le groupe qui se compose de dicarbonates dialkyliques, et le composé (b) est sélectionné parmi le groupe qui se compose d'eau et d'alcools, dans lequel la proportion en composé (b) se situe à pas plus de 2 pour cent en poids, par rapport au poids total du mélange (I).

5. Mélange selon la revendication 4, **caractérisé en ce que** le composé (a) est une β-propiolactone.

6. Mélange selon une des revendications 4, **caractérisé en ce que** le mélange contient un polymère soluble dans le monomère polymérisable.

7. Mélange selon une des revendications 4 à 6, **caractérisé en ce que** le mélange contient un polymère insoluble dans le monomère polymérisable.
